(19) **Europäisches Patentamt**
**European Patent Office**
**Office européen des brevets**

(11) **EP 1 509 547 B1**

(12) **EUROPEAN PATENT SPECIFICATION**

(45) Date of publication and mention
of the grant of the patent:
**29.10.2008 Bulletin 2008/44**

(51) Int Cl.:
***C07K 16/02*** (2006.01)  ***C07K 16/28*** (2006.01)
***A23K 1/00*** (2006.01)  ***A61K 39/395*** (2006.01)

(21) Application number: **03740187.4**

(22) Date of filing: **05.06.2003**

(86) International application number:
**PCT/EP2003/005896**

(87) International publication number:
**WO 2003/104281 (18.12.2003 Gazette 2003/51)**

(54) **ANTOBODIES TO ADIPOSE TISSUES**

ANTIKÖRPER GEGEN FETTGEWEBE

ANTICORPS CONTRE DES CORPS ADIPEUX

(84) Designated Contracting States:
**AT BE BG CH CY CZ DE DK EE ES FI FR GB GR HU IE IT LI LU MC NL PT RO SE SI SK TR**

(30) Priority: **05.06.2002 GB 0212876**

(43) Date of publication of application:
**02.03.2005 Bulletin 2005/09**

(73) Proprietor: **Walcom Animal Science (I.P.4) Limited Kowloon, Hong Kong (CN)**

(72) Inventors:
• **CHI, Francis,
Unit 714
Tsimshatsui,
Kowloon,
Hong Kong (CN)**
• **LU, Tian Shui,
Unit 714
Tsimshatsui,
Kowloon,
Hong Kong (CH)**

(74) Representative: **Arends, William Gerrit et al
Marks & Clerk
90 Long Acre
London
WC2E 9RA (GB)**

(56) References cited:
WO-A-92/16561  WO-A-93/06131
WO-A-98/03081  WO-A-99/08708
US-A- 5 102 658  US-A1- 2002 004 045

• FLINT D J: "Immunological manipulation of adiposity" BIOCHEMICAL SOCIETY TRANSACTIONS, COLCHESTER, ESSEX, GB, vol. 24, no. 2, 1 May 1996 (1996-05-01), pages 418-422, XP002092732 ISSN: 0300-5127
• FLINT DAVID J: "Immunological manipulation of adiposity" PROCEEDINGS OF THE NUTRITION SOCIETY, LONDON, GB, vol. 51, 1992, pages 433-439, XP002086853 ISSN: 0029-6651

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

**Description**

Field of Invention

The present invention relates to a method of producing antibodies that bind adipose tissues and in particular polyclonal antibodies that bind to characterizing components of the plasma membrane in adipose tissues in animals (e.g. farm animals) and/or humans. The present invention also relates to antibodies obtainable according to the method, use of the antibodies, a feed additive comprising the antibodies, a medicament comprising the antibodies, and a method of modulating content of adipose tissues in the body of a target animal in need of the antibodies.

Background of the Invention

In animal farming, one of the main objectives is to increase the growth rate of the farm animals such that under generally the same conditions of husbandry, the animals will grow faster and as such productivity of the animal farm can be increased. In the past, before modern technology has been adopted in animal farming, farmers would normally simply feed the animals with more food in the hope that higher food consumption would cause the animals to grow and increase in weight faster. However, there is a limit that such method can help in increasing the body weight of the animals. Besides, the drawback is that this method would increase the total consumption of animal feed and accordingly undesirably translate to higher operation costs.

Another method to promote growth in farm animals is to administer growth hormones to the animals. This method is however undesirable for a number of reasons. Firstly, growth hormones from different animals are seldom homogenous and different mammalian animals, for example, only react to certain types of specific growth hormones. Since suitable exogenous growth hormones are normally extracted from pituitary glands, it is rather difficult and uneconomical to prepare sufficient quantity of suitable exogenous growth hormones for use on a large-scale application. Although exogenous growth hormones can now be prepared using DNA recombinant technology, exogenous growth hormones manufactured by such method are still rather expensive. Secondly, the administration of exogenous growth hormones into farm animals is normally performed by direct injection, which is inevitably rather costly and difficult to administer in a large farm with animals in tens of thousands. Thirdly, it is rather difficult to control the dose administered to produce precisely the desired effect, and an overdose of exogenous growth hormones is likely to be harmful to the animals. Fourthly, residuals of these exogenous growth hormones may be passed to the meat products and subsequently to humans through consumption thereof. Further studies in this regard are required although some scientists are concerned about the negative side effects of these exogenous growth hormones to humans.

Various feed additives have also been proposed to be added to animal feed such that animals fed with these feed will grow faster. Unfortunately, regardless of which of the above methods is used, it is often the case that a relatively large percentage of the increased body weight results from an increase in fat content and not from lean muscle content. This problem is particularly prominent in swine although other farm animals experience similar problems. As humans have become more health conscious nowadays, there is little demand, if any, for meat products having a high fat content. There is therefore a growing demand for meat products having as low a fat content as possible (i.e. high content of lean muscle).

Numerous methods have been proposed to cause farm animals to develop with higher muscle content. A very old method is to raise the animals in an open or semi-open farm such that the animals would have more opportunity to exercise such that the fat content in their body may be reduced. However, this method is nearly impossible to carry out in practice in modern farms wherein space is at a premium. Besides, this method is rather unpredictable. Animals subjected to this method may still have a rather high fat content in their body.

In US 2002/004045 a method is described to decrease abdominal adipose and peripheral adipose depots in birds by in-ovo inoculation of avian antibodies against adipocyte or and specific adipocyte antigen determinants to 5-28 day-old embryos, or by orally feeding newborn mammals (within 24 hours of birth) with antibodies against adipocyte or and specific adipocyte antigen determinants. US5102658 discusses a method of preventing, controlling or reducing adiposity in which an animal or human being is treated with an effective amount of an antibody to an adipocyte present respectively in said animal or human being. WO9306131 and WO 92/16561 describe antigens present in the plasma membrane of mature porcine white adipocytes or mature ovine white adipocytes respectively, which are not present in porcine liver, kidney, spleen, brain, cardiac muscle, skeletal muscle or lung or in porcine erythrocytes, which react with antisera raised against said adipocytes, and antibodies thereto are useful for the reduction of fat in pigs or sheep respectively.

There continues to exist a need for a substance for regulating and reducing the fat content in farm animals. Preferably, the substance should be easy to administer and natural, and should not have any side effects similar to those caused by artificial or exogenous growth hormones. In other words, the substance should be safe to administer. A substance, which works in farm animals, should preferably also work in humans with modifications.

It is thus an object of the present invention in which the above issues are addressed, or at least to provide a useful alternative to the public.

Summary of Invention

According to a first aspect of the present invention, there is provided a non-therapeutic method of reducing a content of adipose tissues in the body of a target animal using three animals, a source animal, an egg-laying animal and the target animal, the method comprising the steps (i) preparing an antigen from adipose tissues of said source animal; (ii) administering said antigen to said egg-laying animal; (iii) allowing antibodies to be produced by said egg-laying animal in responsive to said antigen; (iv) obtaining said antibodies from eggs of said egg-laying animal; (v) providing an ingestible composition including an effective amount of said antibodies; and (vi) and administering the ingestible composition including an effective amount of said antibodies to said target animal by ingestion, wherein said source animal and said target animal are of different species from said egg-laying animal.

The antibodies are to be used to bind characterizing components of plasma membrane of the adipose tissues of the target animal. In particular, the antibodies may bind granular viscosity proteins and/or fiber viscosity proteins of the adipose tissues of the target animal.

The antibodies suitably specifically bind to the characterizing components. By specifically binding to the charactering components, it means that the antibodies have low, or no noticeable, cross-reactivity.

The method comprises a step of causing production of the antibodies from within the body of the egg-laying animal. The method advantageously comprises a step of causing deposition of the antibodies to the eggs of the egg-laying animal. In particular, the step of obtaining the antibodies from the eggs may comprise a step of isolating the antibodies from the egg yolk of the eggs.

The antigen suitably comprises plasma membrane, its adipocyte plasma membrane surface proteins, or fragments thereof, of the adipose tissues of the source animal.

The antibodies are preferably polyclonal antibodies.

Preferably, the source animal and the egg-laying animal belong to distinctly different species. The egg-laying animal is preferably an avian animal and the source animal may be a non-avian animal. In particular, the egg-laying animal may be a hen and the source animal may be a mammal such as a swine.

Preferably, the target animal and the egg-laying animal belong to different species. More preferably, the target animal and the egg-laying animal belong to distinctly different species. By distinctly different species, it means they are phylogenetically distinct. For example, a swine is a mammal and a hen is an avian, and they are distinctly different species.

Preferably, the target animal and the source animal belong to a same species. Alternatively, the target animal and the source animal belong to closely related species. By closely related species, it means that they are phylogenetically related. For example, a swine and a rat are closely related species and they are both mammals and the antibodies produced in response to the antigen prepared from the adipose tissues of a swine would work sufficiently well in modulating the content of the adipose tissues in a rat.

In one embodiment, the target animal is a farm animal.

In particular, the source animal and/or said target may a swine. The fat content of a swine is often higher than other farm animals and there may be a significant reduction of adipose tissues in the swine administered with the antibodies. However, the target animal may be any other animal which requires modulation of its adipose tissues. For example, the target animal may be a cow for producing leaner beef.

After the method one or all of the animals may be sacrificed.

Also disclosed herein are antibodies obtainable according to the method defined above.

Also disclosed herein is a feed additive comprising an effective amount of antibodies defined above. In particular, the feed additive may be adapted to lower the content of the adipose tissues in the target animal.

The method preferably comprises a step of administering the antibodies via ingestion. Alternatively, the method comprises a step of administering the antibodies via injection.

The antigen is prepared from adipose tissues of a source animal. The antigen comprises plasma membrane and/or its adipocyte plasma membrane surface proteins of the adipose tissues of the source animal.

Also disclosed herein is a feed additive comprising an effective amount of the antibodies defined above. The feed additive is adapted to lower the content of the adipose tissues in the target subject.

Also disclosed herein is a method of modulating content of adipose tissues in the body of a target subject which is a farm animal or a patient in need of antibodies comprising a step of administering a pharmaceutically effective amount of the antibodies that specifically bind the adipose tissues in the target subject.

Also disclosed herein is a method of manufacturing a composition comprising a pharmaceutically effective amount of the antibodies defined above, comprising a step of obtaining the antibodies from eggs of an egg-laying animal. The method preferably comprises a step of allowing deposition of the antibodies to the eggs of the egg-laying animal.

The method preferably comprises a step of causing production of the antibodies from within the body of the egg-laying animal. In particular, the method comprises a step of causing production of the antibodies in the egg-laying animal in response to an antigen prepared from adipose tissues of a source animal. The method comprises a step of administering the antigen to the egg-laying animal.

Detailed Description of the Invention

A biological substance (e.g. growth hormone) may be produced and extracted from the pituitary glands of a "production" animal. By production animal it means the animal is used as a machinery to produce the desired biological substance. Depending on the type or nature of the biological substance, they may actually be obtained or isolated using different methods. For instance, if the biological substance is a growth hormone which is present in the colostrum of cow milk in a production animal, an appropriate isolation procedure of the growth hormone therefrom is to be performed. Alternatively, if the growth hormone is present in the blood serum in a production animal, an alternative suitable isolation procedure of the growth hormone therefrom is to be performed. However, whichever artificial isolation procedure is used, it has been found that isolation of a sufficient quantity of biological substance of interest for commercial use from an animal source is very difficult. The difficulty arises firstly because the quantity of biological substance produced is usually very small. Secondly, isolation of biological substance from the animal is very costly. The same difficulty similarly exists in the extraction or isolation of specific antibodies of interest from a production animal.

In the present invention, it is demonstrated that antibodies prepared in accordance with the present invention when administered to a target animal, which may be a farm animal, reduce or at least modulate the overall fat content in its body to a more desired level and thus produce leaner meat. When the present invention is applied for use in humans, the target animal means a patient in need of the antibodies.

Generally, adipose tissues are firstly removed from a source animal. Plasma membrane of the adipose tissues is then isolated from the adipose tissues. The isolated plasma membrane includes all its adipocyte plasma membrane proteins and recognition sites such as granular and fiber viscosity proteins. The isolated plasma membrane is used to prepare a substance for use as an antigen. The substance is preferably in a form suitable for injection and is engineered to have an immunologically effective concentration of the antigen which is adapted to elicit a desired immunological response in a production animal.

In the present invention, the substance is administered to the production animal which is an egg-laying animal such as a hen. The use of hen as a production animal is particularly preferable because a hen normally produces more eggs than other egg-laying fowl. For instance, an average hen in a commercial farm can often lay as many as 200 to 300 eggs per year. The amount of egg yolk produced by a hen is accordingly very significant. However, other egg-laying animals such as ducks may also be used.

Once the substance containing the antigen is administrated to the production animal such as by injection, the body of the production animal will react and initiate an immune response to the antigen by producing antibodies. As described above, the antigen of the administered substance actually comprises the plasma membrane of the adipose tissues, or fragments thereof, from the source animal. The antibodies produced by the egg-laying animal are thus polyclonal and adapted to bind various different characterizing components, i.e. the adipocyte plasma membrane proteins of the plasma membrane. During the research and development of the present invention, it has been identified that a relatively significant amount of the antibodies produced within the body of the production animal are deposited in the eggs which are subsequently laid by the production animal. It has further been identified that the egg yolk of the eggs has a much higher concentration of the antibodies than the egg white indicating that there is a preferential deposition of the antibodies in the egg yolk. In other words, the problem of producing and isolating a biologically useful substance from an animal source is addressed in the context of the present invention. In particular, the eggs can be seen as a warehouse from which the antibodies of interest can be retrieved relatively easily. It is also for this reason that a hen is preferably used as a production animal because of the relatively large number of eggs that can be produced therefrom.

The produced antibodies can then be isolated from the egg yolk. Alternatively, the raw egg yolk containing the antibodies may be used directly or after processing such as by subjecting it to desiccation to form egg yolk powder. An effective amount of the isolated antibodies, the raw or processed egg yolk containing the antibodies is then administered to a target animal. One main application of the present invention is intended to be in animal farming and in this case the target animal may be a farm animal.

When administered to the target animal, the antibodies will bind to characterizing structures or domains (e.g. the surface proteins of cells in the target animal) which are similar to the adipocyte plasma membrane proteins of the adipose tissues of the source animal. For instance, if the source animal is a swine and the target animal belong to the same species of swine, the administered antibodies will bind to the adipocyte plasma membrane proteins of the adipose tissues in the body of the target animal and interfere with the physiological development of its adipose tissues. It has been identified during the research and development of the present invention that such binding and/or interference significantly decrease the content of adipose tissues in the target animal both in terms of its weight percentage and absolute weight.

As indicated above, the source animal and the target animal may belong to the same species of animals. The more closely related of the source animal and the target animal are, the more effective the produced antibodies for targeting adipose tissues of the target animal and eventually reducing or at least modulating the fat content in the body of the target animal. However, the source animal and the target animal need not belong to identical species. For example, the source animal may be a cow but the target animal may be a swine. Since both cows and swine are mammals, their adipose tissues and in particular the plasma membrane thereof have more resemblance than between for example the adipose tissues of an avian and a mammal. In summary, the more closely related the source animal and target animal are, the more effective the produced antibodies are in binding, interfering, modulating and/or reducing the fat content in the body of the target animal.

It is however to be noted that the source and production animals should preferably be sufficiently different. Otherwise, the substance containing the antigen administered to the production animal would not elicit an effective immunological response to produce a sufficient amount of antibodies of interest. For instance, if the source animal is a duck, the antigen prepared from its adipose tissues will elicit a relatively low immunological response in a hen since both the duck and the hen are avian animals and their adipose tissues are relatively similar. The source animal and the production animal should be different because an animal will not readily produce antibodies to antigen that it considers to be "self".

The present invention is described in further detail by way of the following experiments.

## EXPERIMENTS

### Experiment I: Procedures for Producing Antibodies to Adipose Tissues of Swine in Hens

A. Isolation of plasma membrane from adipose tissues of a source animal

Adipose tissues were removed from the back of a source animal. The source animal used in the experiment was an Erhualian pig. The adipose tissues were treated and homogenized in an extraction medium at around 37°C in a Waring blender at 2000 rpm for 5 min and then treated with ultrasound for 10 minutes. The extraction medium was made of 0.25M of sucrose, 0.01M of $Na_2HP0_4$, 0.002M EDTA, 0.2mM PMSF and adjusted to pH7.4 at 40°C. The homogenate was then centrifuged at 5000 rpm for 30 minutes at 37°C to separate the triglyceride from the other components.

The supernatant containing the triglyceride was then removed after centrifugation and the remainder, i.e. the infranatant, was subjected to centrifugation at 10000 rpm for 30 min at 4°C. The supernatant thereof was then subjected to centrifugation at 10000 rpm for 30 minutes at 4°C and the supernatant was retained. The supernatant was then subjected to centrifugation at 38000 rpm for 1 hour at 4°C. The plasma membrane including its adipocyte membrane proteins from the adipose tissues was obtained. The membrane proteins were then stored at -20°C until they were used.

B. Production of antibodies to pig adipocyte plasma membrane and its proteins

The plasma membrane obtained from the above procedure was used to prepare an antigen to elicit immune response from a production animal. In this experiment, egg-laying hens were used as the production animal. In the experiment, an initial injection comprising the antigen was prepared to contain approximately 80μg of the plasma membrane and its proteins initially suspended in 0.5ml of complete Freund's adjuvant. A second injection comprising the same antigen suspended in incomplete Freund's adjuvant for boosting the immune response was subsequently administered also by direct injection. Each round of administration was performed in at least 20 different intercutaneomucous sites of the hens at intervals of once every four weeks. After the third and fourth booster injections, egg yolk was subsequently obtained from eggs laid by the hens. Antibody responses of the egg yolk were then assessed.

C. Enzyme immunoassay of egg yolk antibodies to plasma membrane of pig adipocytes

The egg yolk antibodies were prepared and screened for antibody titer against a suitable adipocyte plasma membrane, and for cross reactivity with liver, kidney, red blood cells and skeletal muscle by ELISA. 100μl of the plasma membrane containing 0.25μg of the adipocyte plasma membrane proteins in carbonate-buffered solution was coated onto each well of 96-well polystyrene plates. The plates were kept overnight at 4°C in a humidified chamber. The wells were then emptied and blocked with PBS containing 0.05% Tween 20 for three times. 100μl of the egg yolk diluted in PBST was added to each well. The plates were kept for 1 hour at 37°C and subjected to washing with PBST for three times. 100μl of rabbit anti-chicken 1gG HRP conjugate diluted to 1:5000 in PBST was added to each well. The plates were incubated for 1 hour at 37°C. The plates were washed three times with PBST. 100μl of O-phenylenediamin (OPD) substrate (1.5mg/ml) was then added to each well. The plates then were incubated at 37°C for 5 to 10 minutes, and the reaction in each well was stopped with 50ul of 2M $H_2SO_4$. Absorbance was measured at 490nm using an ELA plate reader. Each assay was performed in duplicate and repeated three times. It was found that the titer of the antibodies in the egg yolk was more than 1:12800 which is considered as a relatively high titer value in the context of the present invention.

Experiment II: Effect of adipose tissues antibodies on body weight of target animal

A. Background

The target animal used in this experiment was laboratory rats. Ninety-six female growing rats were used in the experiment with an average body weight of 140g. The rats were divided equally and randomly into four groups. The rats were kept in sub-groups of three in cages. The rats were fed with regular rat feed. The experiment commenced on 30 September 2001 and ended on 14 December 2001.

B. Procedure

The four groups of rats consist of two test groups and two respective control groups. The two test groups include a first test group in which each rat was subjected to injection of raw egg yolk containing the adipose tissue antibodies subcutaneously in different locations at their back. The antibodies are produced in accordance with the procedures of Experiment I above. In particular, the antibodies are produced in response to the injection of an antigen prepared from the adipose tissues of a pig. The egg yolk adipose tissue antibodies were obtained based on similar procedures described in the above Experiment I. The dose of each injection was 1ml per rat per day. Each round of administration includes one injection each day for four consecutive days. The egg yolk was administered again once a month during the experiment. The titer of the antibodies in the raw egg yolk was more than 1:12800.

The second test group was administered with the same dose, concentration and frequency of the egg yolk adipose tissue antibodies but by oral ingestion instead of injection.

There is a corresponding control group for each of the two test groups of rats. The control groups of rats were administered with regular raw egg yolk.

C. Results

The following tables show the results of the experiment.

TABLE 1: Effects of the egg yolk adipose tissue antibodies on body weight and feed conversion rate ($X \pm SE$)

| | Beginning body weight (g) | Ending body weight (g) | Body weight gain (g) | Food intake (g) | Feed conversion rate |
|---|---|---|---|---|---|
| **First test group (by injection)** | 163.42±2.55 | 297.64±5.23 | 133.91±4.23 | 22.25±0.23 | 6.25±0.20 |
| **First control group (by injection)** | 162.88±2.28 | 289.00±5.33 | 126.62±4.40 | 21.87±0.26 | 6.27±0.30 |
| **Second test group (by ingestion)** | 159.10±2.70 | 281.56±7.43 | 122.25±5.02 | 21.75±0.23 | 5.87±0.22 |
| **Second control group (by ingestion)** | 164.21±2.00 | 292.82±6.54 | 127.18±6.20 | 21.72±0.52 | 6.52±0.21 |

TABLE 2: Effects of the egg yolk adipose tissue antibodies on fat content in various parts of the rat body (X±SE)

| | Omental and mesenteric fat Content (%) | Paramentrial fat Content (%) | Perirenal fat Content (%) | Gastrocnemius fat Content (%) |
|---|---|---|---|---|
| First test group (by injection) | 18.06±0.72Aa | 26.43±1.72Aa | 17.95±1.48Aa | 6.03±0.11a |
| First control group (by injection) | 18.75±0.87Aa | 27.58±1.78Aa | 19.18±1.32Aa | 5.73±0.06b |
| Second test group (by ingestion) | 14.22±1.02Bb | 18.63±1.98Bb | 12.01±1.17Bb | 5.89±0.11 |
| Second control group (by ingestion) | 17.16±1.05a | 24.58±2.24a | 15.32±1.25a | 5.83±0.09 |
| KEY: Values bearing different superscripts are significantly different; A, B means P<0.01; a, b means P<0.05 | | | | |

TABLE 3: Effects of the egg yolk adipose tissue antibodies on level of triglyceride, cholesterol and fatty acids in blood of the rat body (X±SE)

| | Triglyceride (mg/dl) | Total cholesterol (mg/dl) | Total fatty acids (μmol/L) |
|---|---|---|---|
| First test group (by injection) | 33.83±1.70Aa | 61.05±3.56 | 140.69±9.73 |
| First control group (by injection) | 45.42±2.67B | 58.91±2.44 | 135.29±7.31 |
| Second test group (by ingestion) | 32.00±1.60Aa | 61.35±2.61 | 161.21±8.05A |
| Second control group (by ingestion) | 41.20±2.48b | 54.64±4.21 | 121.72±7.47B |
| KEY: Values bearing different superscripts are significantly different; A, B means P<0.01; a, b means P<0.05 | | | |

D. Conclusion and discussion

In Table 1, it is shown that the administration of the antibodies by injection increased the weight gain and the food consumption in the first test group of rats when compared to the corresponding control group by 5.8% and by 1.7% respectively. The feed conversion rate was however decreased by 0.32%. It is also shown that the administration of the antibodies by ingestion when compared to the corresponding control group decreased the weight gain in the second test group of rats by 3.9% and increased the food consumption by 0.14%. The feed conversion rate was decreased by about 10%. The experimental data in relation to the first test and control groups illustrates that the administration of the antibodies by injection increased the body weight slightly although the feed conversion rate was lowered very slightly. A low feed conversion rate means that less amount of feed is required to produce a unit of body weight. The experimental data in relation to the second and test and control groups illustrates that the administration of the antibodies by ingestion decreased the body weight gain slightly and the feed conversion efficiency was substantially decreased by 10%. This is important and demonstrates that the administration of the antibodies through ingestion is more effective in reducing the overall body weight slightly and lowering the feed conversion rate very significantly.

Referring to Table 2, it is shown that the administration of the antibodies by injection caused to the fat content of their omental and mesenteric, paramentrial and perirenal tissues to decrease by 3.7%, 4.2% and 6.4% respectively when compared to the corresponding control groups. However, the fat content of the gastrocnemius was increased by 5.2%. It is also shown that the administration of the antibodies by ingestion very significantly decreased the fat content of their omental and mesenteric, paramentrial and perirenal tissues by 17.1%, 24.2% and 2.16% respectively when compared to the corresponding control group.

As clearly shown in this experiment, the administration of the antibodies by whichever means, injection or ingestion, is generally effective in reducing the fat content in various parts of the body in the animal. In particular, it is shown that administration by way of ingestion is significantly more effective in reducing the general fat content of the animal.

Referring to Table 3, it is shown that the administration of the antibodies by injection caused the level of triglyceride to decrease significantly by 25.5%. The levels of cholesterol and free fatty acids were caused to rise marginally by 3.6% and 4.0% respectively. In relation to the administration of the antibodies through oral ingestion, the level of triglyceride was caused to decrease also significantly by 22.3%. The levels of cholesterol and free fatty acids were caused to increase by 12.3% or 32.4 respectively.

When the data of all three tables are considered together, it is clearly shown that the administration of the antibodies into the animal does reduce the overall fat content in its body and this is supported by the decrease in the overall fat content in the test groups of rats shown in Table 2 and the levels of triglyceride shown in Table 3. In particular, it is shown that administration of the antibodies by means of oral ingestion is more effective when compared to that by direct injection.

The above results are significant in two ways. Firstly, surprisingly, the antibodies produced according to the present invention are more effective when administered orally. This is important because the antibodies can in principle be mixed with a standard feed material in animal farming and as such administration thereof will become very easy, effective and yet can achieve its intended function in reducing fat content. Secondly, there are no observable side effects to the animal. For instance, the overall body weight is not affected in any significant way and yet the fat content is reduced. The feed conversion rate is also slightly improved. In other words, there is less fat content and higher lean meat content in the body of the target animal.

In table 3, it is shown that the level of free fatty acids was increased significantly. This can be explained as follows. Triglyceride is composed of fatty acids and glycerol. When the level of triglyceride (i.e. fat content) is caused to be reduced, the equilibrium is shifted to the right as illustrated below.

$$\texttt{Triglyceride <=> fatty acids + glycerol}$$

$$\rightarrow$$

For this reason, the level of free fatty acids was caused to increase.

Experiment III: Effect of adipose tissues antibodies on body weight of rats

A. Background and Procedure

The target animal used in this experiment was laboratory rat. One hundred fifty female growing rats were used in the experiment with an average body weight of 110g. The rats were randomly divided into five groups (i.e. Groups I to V) in cages with each cage keeping three rats. Group I was the control group and Groups II to V were the test groups. The experiment was preceded by one week feeding the rats with a regular feed. The composition of the regular feed is as follows.

Table 4: Composition of the regular feed

| Ingredients | Wt% |
| --- | --- |
| Proteins | 24.02 |
| Fats | 3.94 |
| | 7.9 |
| Calcium | 1.4 |
| Phosphorus | 0.8 |
| Salts | 1.31 |

During the experiment, the Group I rats were fed with the regular diet. The Groups II to V rats were fed with

8

EP 1 509 547 B1

the regular diet added with different quantities of adipose tissues antibodies prepared in accordance with the method described in Experiment I above. The antibodies are produced in accordance with the procedures of Experiment I above. In particular, the antibodies are produced in response to the injection of an antigen prepared from the adipose tissues of a pig.

The experiment began on 28 November 2002 and ended on 18 February 2003.

B. Results

Table 5: Effect of antibodies on adipose tissues (x±sd)

| Weight\Antibodies in diet | Control | 75ppm | 500ppm | 1000ppm | 6000ppm |
|---|---|---|---|---|---|
| Beginning body weight (g) | 115.19± 6.78 | 115.24± 7.81 | 114.72± 7.54 | 114.81± 6.65 | 115.65± 9.19 |
| Final body weight | 304.13± 14.65 | 302.23± 21.38 | 296.77± 22.68 | 296.66± 23.17 | 304.00± 23.16 |
| Weight gain (g) | 188.94± 16.37 | 187.00± 21.75 | 182.04± 21.52 | 181.68± 25.09 | 188.05± 18.92 |
| P value | | 0.70 | 0.17 | 0.19 | 0.84 |
| Food intake (g/day/rat) | 24.68± 2.18 | 24.42± 2.63 | 23.93± 1.90 | 25.53± 2.17 | 25.90± 1.33 |
| P value | | 0.81 | 0.42 | 0.39 | 0.15 |
| | | | | | |
| Parametrial fat (g) | 6.04± 1.66 | 5.02± 1.72 | 5.33± 1.93 | 5.97± 2.10 | 5.14± 1.79 |
| P value | | 0.025 | 0.137 | 0.887 | 0.053 |
| Parametrial fat index | 19.80± 5.20 | 16.44± 4.96 | 18.11± 6.27 | 20.00± 6.69 | 16.90± 5.24 |
| P value | | 0.014 | 0.266 | 0.898 | 0.041 |
| | | | | | |
| Mesenteric fat (g) | 4.64± 0.86 | 4.28± 0.99 | 4.42± 0.96 | 4.44± 1.16 | 4.15± 1.00 |
| P value | | 0.149 | 0.362 | 0.458 | 0.052 |
| Mesenteric fat index | 15.21± 2.56 | 14.20± 2.80 | 14.98± 2.89 | 14.82± 2.98 | 13.69± 2.74 |
| P value | | 0.155 | 0.739 | 0.588 | 0.033 |
| | | | | | |
| Perirenal fat (g) | 4.80± 1.39 | 4.23± 1.47 | 4.06± 1.37 | 4.01± 1.35 | 3.83± 1.37 |
| P value | | 0.144 | 0.054 | 0.041 | 0.013 |
| Perirenal fat index | 15.79± 4.40 | 13.90± 4.36 | 13.85± 4.54 | 13.55± 4.46 | 12.66± 4.35 |
| P value | | 0.113 | 0.115 | 0.071 | 0.011 |
| | | | | | |
| Celiac fat (g) | 15.48± 3.36 | 13.53± 3.79 | 13.74± 3.80 | 14.21± 4.67 | 13.46± 4.07 |
| P value | | 0.041 | 0.069 | 0.240 | 0.044 |
| Celiac fat index | 50.76± 10.25 | 44.53± 10.67 | 46.77± 12.20 | 47.72± 14.21 | 44.33± 11.94 |
| P value | | 0.026 | 0.182 | 0.352 | 0.032 |
| | | | | | |
| Gastrocnemius (g) | 1.85± 0.11 | 1.85± 0.13 | 1.81± 0.12 | 1.83± | 1.87± 0.17 |

(continued)

| Weight\Antibodies in diet | Control | 75ppm | 500ppm | 1000ppm | 6000ppm |
|---|---|---|---|---|---|
| P value | | 0.946 | 0.319 | 0.521 | 0.561 |
| Gastricnemius muscle index | 6.09± 0.37 | 6.09± 0.37 | 6.10± 0.45 | 6.17± 0.32 | 6.15± 0.41 |
| P value | | 0.995 | 0.973 | 0.428 | 0.582 |
| | | | | | |
| Serum glycerinate (mg/dl) | 56.76± 24.33 | 58.59± 19.15 | n/a | n/a | 44.95± 15.76 |
| P value | | 0.756 | | | 0.040 |
| Serum free fatty acid (umol/l) | 151.08± 79.12 | 163.36± 82.92 | n/a | n/a | 209.45± 125.76 |
| P value | | 0.573 | | | 0.041 |
| | | | | | |
| Serum leptin (ng/ml) | 1.89± 0.66 | 1.73± 0.63 | n/a | n/a | 1.68± 0.52 |
| P value | | 0.29 | | | 0.18 |
| Serum insulin (uU/ml) | 25.78± 7.63 | 20.11± 4.87 | n/a | n/a | 22.49± 5.83 |
| P value | | 0.0017 | | | 0.0882 |

C. Discussion and conclusion

The above results indicate that the rats fed with a diet added with 75ppm antibodies had 16.89% reduction in parametrial fat, 7.76% reduction in mesenteric fat, 11.88% reduction in perirenal fat and 12.60g reduction celiac fat compared with the control rats. The rats fed with the diet added with 75ppm antibodies had no noticeable difference in gastrocnemius muscle weight compared with the control rats.

The above results indicate that the rats fed with a diet added with 6000ppm antibodies had 14.90% reduction in parametrial fat, 10.56% reduction in mesenteric fat, 20.21% reduction in perirenal fat and 13.05g reduction celiac fat compared with the control rats. The rats fed with the diet added with 6000ppm antibodies had 1.08% increase in gastrocnemius muscle weight compared with the control rats. The increase was however insignificant.

The experimental data suggests that the amount of food intake by the different groups of rats was about the same statistically.

The experimental data suggest that the antibodies prepared and administered in accordance with the present invention is effective in modulating and in particular reducing the adipose tissues in a target animal.

Based on the findings of the above experiments, the antibodies when administered in animal farming (e.g. via an animal feed) can produce animals with leaner meat. Among most farm animals for producing meat for human consumption, swine tend to have a rather high fat content. Thus, the present invention is particularly suitable to be applied in raising swine.

When applied for use in humans, the antibodies can be used in the manufacture of a medicament or composition for the non-therapeutic treatment or prevention of obesity and/or related conditions. Alternatively, the antibodies can be added to a food supplement suitable for consumption by humans. A medicament comprising such antibodies may also be produced.

**Claims**

1.   A non-therapeutic method of reducing a content of adipose tissues in the body of a target animal using three animals, a source animal, an egg-laying animal and the target animal, the method comprising the steps of:

(i) preparing an antigen from adipose tissues of said source animal;

(ii) administering said antigen to said egg-laying animal;

(iii) allowing antibodies to be produced by said egg-laying animal in response to said antigen;

(iv) obtaining said antibodies from eggs of said egg-laying animal;

(v) providing an ingestible composition including an effective amount of said antibodies; and

(vi) administering the ingestible composition including an effective amount of said antibodies to said target animal by ingestion, wherein said source animal and said target animal are of different species from said egg-laying animal.

2. A method according to Claim 1 wherein said step of obtaining comprises a step of isolating said antibodies from the egg yolk of said eggs.

3. A method according to Claim 1 or 2 comprising a step of binding said antibodies to characterizing components of plasma membrane of said adipose tissues in said target animal.

4. A method according to Claim 1, 2 or 3 comprising a step of binding said antibodies to granular viscosity proteins of said adipose tissues in said target animal.

5. A method according to Claim 1, 2 or 3 comprising a step of binding said antibodies to fiber viscosity proteins of said adipose tissues in said target animal.

6. A method according to Claim 1 wherein said antigen comprises plasma membrane, its adipocyte plasma membrane surface proteins, or fragments thereof, of said adipose tissues of said source animal.

7. A method according to Claim 1 wherein said antibodies are polyclonal antibodies.

8. A method according to Claim 1 wherein said target animal and said source animal belong to same species.

9. A method according to Claim 1 wherein said target animal is a farm animal.

10. A method according to Claim 1 wherein said source animal and/or said target is a swine.

11. A method according to Claim 1 wherein said target animal is human being.

12. A method according to Claim 1 wherein said egg-laying animal is an avian animal and said source animal is a non-avian animal.

13. A method according to Claim 1 wherein said source animal is a mammal.

14. A method according to Claim 1 wherein one or more of said target animals is sacrificed after said method when said target animal is not a human being.


**Patentansprüche**

1. Nichttherapeutisches Verfahren zur Verminderung eines Fettgewebegehalts im Körper eines Zieltiers unter Verwendung von drei Tieren, eines Quellentiers, eines eierlegenden Tiers und des Zieltiers, wobei das Verfahren die folgenden Schritte aufweist:

(i) Herstellen eines Antigens aus Fettgewebe des Quellentiers;

(ii) Verabreichen des Antigens an das eierlegende Tier;

(ii) Ermöglichen der Produktion von Antikörpern durch das eierlegende Tier als Reaktion auf das Antigen;

(iv) Gewinnen von Antikörpern aus Eiern des eierlegenden Tiers;

(v) Bereitstellen einer einnehmbaren Zusammensetzung, die eine wirksame Menge der Antikörper enthält; und

(vi) Verabreichen der einnehmbaren Zusammensetzung, die eine wirksame Menge der Antikörper enthält, an das Zieltier durch Ingestion, wobei das Quellentier und das Zieltier zu Spezies gehören, die sich von der Spezies des eierlegenden Tiers unterscheiden.

**2.** Verfahren nach Anspruch 1, wobei der Gewinnungsschritt einen Schritt zum Isolieren der Antikörper aus dem Eidotter der Eier aufweist.

**3.** Verfahren nach Anspruch 1 oder 2, das einen Schritt zur Bindung der Antikörper an charakterisierende Komponenten der Plasmamembran der Fettgewebe in dem Zieltier aufweist.

**4.** Verfahren nach Anspruch 1, 2 oder 3, das einen Schritt zur Bindung der Antikörper an granuläre viskose Proteine der Fettgewebe in dem Zieltier aufweist.

**5.** Verfahren nach Anspruch 1, 2 oder 3, das einen Schritt zur Bindung der Antikörper an faserförmige viskose Proteine der Fettgewebe in dem Zieltier aufweist.

**6.** Verfahren nach Anspruch 1, wobei das Antigen eine Plasmamembran aufweist und deren Fettzellenplasmamembranoberflächenproteine aus der Fettgewebe des Quellentiers oder Fragmente davon aufweist.

**7.** Verfahren nach Anspruch 1, wobei die Antikörper polyklonale Antikörper sind.

**8.** Verfahren nach Anspruch 1, wobei das Zieltier und das Quellentier zur gleichen Spezies gehören.

**9.** Verfahren nach Anspruch 1, wobei das Zieltier ein landwirtschaftliches Nutztier ist.

**10.** Verfahren nach Anspruch 1, wobei das Quellentier und/oder das Zieltier ein Schwein ist.

**11.** Verfahren nach Anspruch 1, wobei das Zieltier ein Mensch ist.

**12.** Verfahren nach Anspruch 1, wobei das eierlegende Tier ein vogelartiges Tier und das Quellentier ein nicht vogelartiges Tier ist.

**13.** Verfahren nach Anspruch 1, wobei das Quellentier ein Säugetier ist.

**14.** Verfahren nach Anspruch 1, wobei eines oder mehrere der Zieltiere nach Abschluß des Verfahrens getötet wird, wenn das Zieltier kein Mensch ist.

**Revendications**

**1.** Procédé non thérapeutique de réduction d'une teneur en tissus adipeux dans le corps d'un animal cible en utilisant trois animaux, un animal source, un animal ovipare et l'animal cible, le procédé comprenant les étapes consistant:

    (i) à préparer un antigène à partir de tissus adipeux dudit animal source;
    (ii) à administrer ledit antigène audit animal ovipare;
    (iii) à permettre la production d'anticorps par ledit animal ovipare en réponse audit antigène;
    (iv) à obtenir lesdits anticorps à partir d'oeufs dudit animal ovipare;
    (v) à procurer une composition ingérable incluant une quantité efficace desdits anticorps; et
    (vi) à administrer la composition ingérable incluant une quantité efficace desdits anticorps audit animal cible par ingestion,

dans lequel ledit animal source et ledit animal cible sont d'espèces différentes dudit animal ovipare.

**2.** Procédé selon la revendication 1, dans lequel ladite étape d'obtention comprend une étape consistant à isoler lesdits anticorps à partir du jaune d'oeuf desdits oeufs.

**3.** Procédé selon la revendication 1 ou 2, comprenant une étape consistant à lier lesdits anticorps à des constituants caractérisants de la membrane plasmatique desdits tissus adipeux dans ledit animal cible.

**4.** Procédé selon la revendication 1, 2 ou 3, comprenant une étape consistant à lier lesdits anticorps à des protéines de viscosité granulaire desdits tissus adipeux dans ledit animal cible.

5. Procédé selon la revendication 1, 2 ou 3, comprenant une étape consistant à lier lesdits anticorps à des protéines de viscosité fibreuse desdits tissus adipeux dans ledit animal cible.

6. Procédé selon la revendication 1, dans lequel ledit antigène comprend une membrane plasmatique, ses protéines de surface de membrane plasmatique adipocytes, ou des fragments de celle-ci, desdits tissus adipeux dudit animal source.

7. Procédé selon la revendication 1, dans lequel lesdits anticorps sont des anticorps polyclonaux.

8. Procédé selon la revendication 1, dans lequel ledit animal cible et ledit animal source appartiennent à la même espèce.

9. Procédé selon la revendication 1, dans lequel ledit animal cible est un animal de ferme.

10. Procédé selon la revendication 1, dans lequel ledit animal source et/ou ledit animal cible est/sont un porc.

11. Procédé selon la revendication 1, dans lequel ledit animal cible est un être humain.

12. Procédé selon la revendication 1, dans lequel ledit animal ovipare est un animal avien et ledit animal source est un animal non avien.

13. Procédé selon la revendication 1, dans lequel ledit animal source est un mammifère.

14. Procédé selon la revendication 1, dans lequel un ou plusieurs desdits animaux cibles est (sont) sacrifié(s) après ledit procédé lorsque ledit animal cible n'est pas un être humain.

**REFERENCES CITED IN THE DESCRIPTION**

**Patent documents cited in the description**

- US 2002004045 A
- US 5102658 A
- WO 9306131 A
- WO 9216561 A